(19)

Europäisches
Patentamt
European
Patent Office
Office européen
des brevets

(11) **EP 1 299 421 B1**

(12) **EUROPEAN PATENT SPECIFICATION**

(45) Date of publication and mention
of the grant of the patent:
**25.03.2009 Bulletin 2009/13**

(21) Application number: **01962800.7**

(22) Date of filing: **29.06.2001**

(51) Int Cl.:
**C07K 16/24** (2006.01) **C12N 15/13** (2006.01)
**C12N 15/63** (2006.01) **C12P 21/08** (2006.01)
**A61K 39/395** (2006.01)

(86) International application number:
**PCT/EP2001/007468**

(87) International publication number:
**WO 2002/002640 (10.01.2002 Gazette 2002/02)**

(54) **ANTIBODIES TO HUMAN MCP-1**

ANTIKÖRPER GEGEN MENSCHLICHES MCP-1

ANTICORPS ANTI-MCP-1 HUMAIN

(84) Designated Contracting States:
**AT BE CH CY DE DK ES FI FR GB GR IE IT LI LU
MC NL PT SE TR**
Designated Extension States:
**RO SI**

(30) Priority: **30.06.2000 GB 0016138**

(43) Date of publication of application:
**09.04.2003 Bulletin 2003/15**

(73) Proprietors:
• **NOVARTIS AG**
**4056 Basel (CH)**
Designated Contracting States:
**BE CH CY DE ES FI FR GB GR IE IT LI LU MC NL
PT SE TR**
• **Novartis Pharma GmbH**
**1230 Wien (AT)**
Designated Contracting States:
**AT**

(72) Inventors:
• **HIESTAND, Peter**
**CH-4123 Allschwil (CH)**
• **HOFSTETTER, Hans**
**CH-4125 Riehen (CH)**
• **PAYNE, Trevor, Glyn**
**CH-3005 Berne (CH)**

• **URFER, Roman**
**CH-4056 Basel (CH)**
• **DI PADOVA, Franco, E.**
**CH-4127 Birsfelden (CH)**

(74) Representative: **Gros, Florent et al**
**Novartis AG**
**Corporate Intellectual Property,**
**Patent & Trademark Department CH**
**4002 Basel (CH)**

(56) References cited:
**US-A- 5 985 279**

• **BADIE BEHNAM ET AL: "In vitro modulation of
microglia motility by glioma cells is mediated by
hepatocyte growth factor/scatter factor."
NEUROSURGERY (BALTIMORE), vol. 44, no. 5,
May 1999 (1999-05), pages 1077-1083,
XP000984052 ISSN: 0148-396X**
• **BADOLATO RAFFAELE ET AL: "Interleukin-15
(IL-15) induces IL-8 and monocyte chemotactic
protein 1 production in human monocytes."
BLOOD, vol. 90, no. 7, 1997, pages 2804-2809,
XP002188285 ISSN: 0006-4971**
• **Little, M. et al (2000) Immunology Today 21:
364-370**

**Description**

This invention relates to antibodies to human monocyte chemoattractant protein (MCP)-1 and to the use of such antibodies for the treatment of diseases and disorders which involve migration and activation of monocytes and T-cells, e.g. inflammatory diseases.

Published Japanese patent application JP 05276986, (Sumitomo Electric Co.) describes the preparation of rodent monoclonal antibodies to human MCP-1 useful for determining MCP-1 and for treating and diagnosing diseases which involve macrophage infiltration. Published Japanese patent application JP 09067399 (Mitsui Toatsu) describes the preparation of human monoclonal antibodies to human MCP-1 from EBV transformed human peripheral blood cells for use in the treatment of inflammation. Published Japanese patent application JP 11060502 (Teijin) describes use of an MCP-1 inhibitor, in particular a human anti-MCP-1 antibody for treatment of cerebral infarction.

We have now prepared improved antibodies to human MCP-1 for use in the treatment of diseases and disorders which involve migration and activation of monocytes and T-cells.

Accordingly the invention provides an MCP-1 binding molecule which comprises (a) an antigen binding site comprising an immunoglobulin heavy chain variable domain ($V_H$) which comprises in sequence hypervariable regions CDR1, CDR2 and CDR3, said CDR1 having the amino acid sequence His-Tyr-Trp-Met-Ser, said CDR2 having the amino acid sequence Asn-Ile-Glu-Gln-Asp-Gly-Ser-Glu-Lys-Tyr-Tyr-Vaf-Asp-Ser-Val-lys-Gly, and said CDR3 having the amino acid sequence Asp-Leu-Glu-Gly-Leu-His-Gly-Asp-Gly-Tyr-Phe-Asp-Leu; and (b) an immunoglobulin light chain variable domain ($V_L$) which comprises in sequence hypervariable regions CDR1', CDR2' and CDR3', said CDR1' having the amino acid sequence Arg-Ala-Ser-Gln-Gly-Vaf-Ser-Ser-Ala-Leu-Ala, said CDR2' having the amino acid sequence Asp-Ala-Ser-Ser-Leu-Glu-Ser, and said CDR3' having the amino acid sequence Gln-Gln-Phe-Asn-Ser-Tyr-Pro.

Unless otherwise indicated, any polypeptide chain is herein described as having an amino acid sequence starting at the N-terminal extremity and ending at the C-terminal extremity.

When the antigen binding site comprised both the $V_H$ and $V_L$ domains, these may be located on the same polypeptide molecule or, preferably, each domain may be on a different chain, the $V_H$ domain being part of an immunoglobulin heavy chain or fragment thereof and the $V_L$ being part of an immunoglobulin light chain or fragment thereof.

By "MCP-1 binding molecule" is meant any molecule capable of binding to the MCP-1 antigen either alone or associated with other molecules. The binding reaction may be shown by standard methods (qualitative assays) including, for example, a bioassay for determining the inhibition of MCP-1 binding to its receptor, i.e. the chemokine receptor (CCR)-2, e.g. CCR2B, or any kind of binding assays, with reference to a negative control test in which an antibody of unrelated specificity, but preferably of the same isotype, is used. Advantageously, the binding of the MCP-1 binding molecules of the invention to MCP-1 may be shown, for instance, in a BIAcore assay.

Examples of antigen binding molecules include antibodies as produced by B-cells or hybridomas and chimeric, CDR-grafted or human antibodies or any fragment thereof, e.g. $F(ab')_2$ and Fab fragments.

A single chain antibody consists of the variable domains of the heavy and light chains of an antibody covalently bound by a peptide linker usually consisting of from 10 to 30 amino acids, preferably from 15 to 25 amino acids. Therefore, such a structure does not include the constant part of the heavy and light chains and it is believed that the small peptide spacer should be less antigenic than a whole constant part. By "chimeric antibody" is meant an antibody in which the constant regions of heavy or light chains or both are of human origin while the variable domains of both heavy and light chains are of non-human (e.g. murine) origin or of human origin but derived from a different human antibody. By "CDR-grafted antibody" is meant an antibody in which the hypervariable regions (CDRs) are derived from a donor antibody, such as a non-human (e.g. murine) antibody or a different human antibody, while all or substantially all the other parts of the immunoglobulin e.g. the constant regions and the highly conserved parts of the variable domains, i.e. the framework regions, are derived from an acceptor antibody, e.g. an antibody of human origin. A CDR-grafted antibody may however contain a few amino acids of the donor sequence in the framework regions, for instance in the parts of the framework regions adjacent to the hypervariable regions. By "human antibody" is meant an antibody in which the constant and variable regions of both the heavy and light chains are all of human origin, or substantially identical to sequences of human origin, not necessarily from the same antibody and includes antibodies produced by mice in which the murine immunoglobulin variable and constant part genes have been replaced by their human counterparts, e.g. as described in general terms in EP 0546073 B1, USP 5545806, USP 5569825, USP 5625126, USP 5633425, USP 5661016, USP 5770429, EP 0 43 8474 B1 and EP 0 463151 B1.

Particularly preferred MCP-1 binding molecules of the invention are human antibodies especially the AAV293, AAV294 and ABN912 antibodies as hereinafter described in the Examples. (The AAV293 antibody is a human IgG3/k antibody and ABN912 is a human IgG4/k antibody, but are essentially identical in other respects. The AAV294 antibody is a human IgG1/κ antibody which has variable domains which are identical to those of AAV293 except for single amino acid changes in FR1, CDR2 and FR3 of $V_H$ and CDR1' and FR3' of $V_L$, as hereinafter described in the Examples.)

Thus in preferred chimeric antibodies the variable domains of both heavy and light chains are of human origin, for instance those of the ABN912 antibody which are shown in Seq. Id. No. 1 and Seq. Id. No. 2. The constant region domains preferably also comprise suitable human constant region domains, for instance as described in "Sequences of Proteins of Immunological Interest", Kabat E.A. et al, US Department of Health and Human Services, Public Health Service, National Institute of Health

Hypervariable regions may be associated with any kind of framework regions, though preferably are of human origin. Suitable framework regions are described in Kabat E.A. et al, ibid. The preferred heavy chain framework is a human heavy chain framework, for instance that of the ABN912 antibody which is shown in Seq. Id. No. 1. It consists in sequence of FR1, FR2, FR3 and FR4 regions. In a similar manner, Seq. Id. No. 2 shows the preferred ABN912 light chain framework which consists, in sequence, of FR1', FR2', FR3' and FR4' regions. Alternative framework regions, preferably human framework regions, may be used to those shown in Seq. Id. No. 1 and Seq. Id. No. 2, for example as described in Kabat et al. Ibid. A few amino acid residues of the framework regions, in particular in the parts of the framework adjacent to the hypervariable regions, may differ from those of the relevant defined framework region, e.g. to influence binding properties.

Accordingly, the invention also provides an MCP-1 binding molecule which comprises at least one antigen binding site comprising either a first domain having an amino acid sequence substantially identical to that shown in Seq. Id. No. 1 starting with amino acid at position 1 and ending with amino acid at position 122 or a first domain as described above and a second domain having an amino acid sequence substantially identical to that shown in Seq. Id. No. 2, starting with amino acid at position 1 and ending with amino acid at position 109.

Monoclonal antibodies raised against a protein naturally found in all humans are typically developed in a non-human system e.g. in mice. As a direct consequence of this, a xenogenic antibody as produced by a hybridoma, when administered to humans, elicits an undesirable immune response which is predominantly mediated by the constant part of the xenogenic immunoglobulin. This clearly limits the use of such antibodies as they cannot be administered over a prolonged period of time. Therefore it is particularly preferred to use single chain, single domain, chimeric, CDR-grafted, or especially human antibodies which are not likely to elicit a substantial allogenic response when administered to humans.

In view of the foregoing, a more preferred MCP-1 binding molecule of the invention is selected from a human anti MCP-1 antibody which comprises at least

a) an immunoglobulin heavy chain or fragment thereof which comprises (i) a variable domain comprising in sequence the hypervariable regions CDR1, CDR2 and CDR3 and (ii) the constant part of fragment thereof of a human heavy chain; said CDR1 having the amino acid sequence His-Tyr-Trp-Met-Ser, said CDR2 having the amino acid sequence Asn-Ile-Glu-Gln-Asp-Gly-Ser-Glu-Lys-Tyr-Tyr-Val-Asp-Ser-Val-Lys-Gly, and said CDR3 having the amino acid sequence Asp-Leu-Glu-Gly-Leu-His-Gly-Asp-Gly-Tyr-Phe-Asp-Leu and

(b) an immunoglobulin light chain or fragment thereof which comprises (i) a variable domain comprising the CDR3' hypervariable region and optionally also the CDR1', CDR2' hypervariable regions and (ii) the constant part or fragment thereof of a human light chain, said CDR1' having the amino acid sequence Arg-Ala-Ser-Gln-Gly-Val-Ser-Ser-Ala-Leu-Ala, said CDR2' having the amino acid sequence Asp-Ala-Ser-Ser-Leu-Glu-Ser, and said CDR3' having the amino acid sequence Gln-Gln-Phe-Asn-Ser-Tyr-Pro.

Alternatively, an MCP-1 binding molecule of the invention may be selected from a single chain binding molecule which comprises an antigen binding site comprising

a) a first domain comprising in sequence the hypervariable regions CDR1, CDR2 and CDR3, said hypervariable regions having the amino acid sequences as shown in Seq. Id No.1,
b) A second domain comprising the hypervariable regions CDR1', CDR2' and CDR3', said hypervariable regions having the amino acid sequences as shown in Seq.Id. No.2 and
c) a peptide linker which is bound either to the N-terminal extremity of the first domain and the C-terminal extremity of the second domain or to the C-terminal extremity of the first domain and to the N-terminal extremity of second domain.

As it is well known, minor changes in an amino acid sequence such as deletion, addition or substitution of one, a few or even several amino acids may lead to an allelic form of the original protein which has substantially identical properties.

Thus, by the term "direct equivalents thereof' is meant either any single domain MCP-1 binding molecule (molecule X).

(i) in which the hypervariable regions CDR1, CDR2 and CDR3 taken as a whole are at least 80% homologous, preferably at least 90% homologous, more preferably at least 95% homologous to the hypervariable regions as shown in Seq. Id. No. 1 and,

(ii) which is capable of inhibiting the binding of MCP-1 to its receptor substantially to the same extent as a reference molecule having framework regions identical to those of molecule X but having hypervariable regions CDR1, CDR2 and CDR3 identical to those shown in Seq. Id. No. 1

or any MCP-1 binding molecule having at least two domains per binding site (molecule X')

(i) in which the hypervariable regions CDR1, CDR2, CDR3, CDR1', CDR2' and CDR3' taken as a whole are at least 80% homologous, preferably at least 90% homologous, more preferably at least 95% homologous, to the hypervariable regions as shown in Seq. Id. No. 1 and 2 and

(ii) which is capable of inhibiting the binding of MCP-1 to its receptor substantially to the same extent as a reference molecule having framework regions and constant parts identical to molecule X', but having hypervariable regions CDR1, CDR2, CDR3, CDR1', CDR2' and CDR3', identical to those shown in Seq. Id. No. 1 and 2.

In the present description amino acid sequences are at least 80% homologous to one another if they have at least 80% identical amino acid residues in a like position when the sequence are aligned optimally, gaps or insertions in the amino acid sequences being counted as non-identical residues.

The inhibition of the binding of MCP-1 to its receptor may be conveniently tested in various assays including such assays are described hereinafter in the text. By the term "to the same extent" is meant that the reference and the equivalent molecules exhibit, on a statistical basis, essentially similar MCP-1 binding inhibition curves in one of the assays referred to above. For example, the MCP-1 binding molecules of the invention typically have $IC_{50}$s for inhibition of the binding of MCP-1 to its receptor (CCR2B) which are within +/- x 5 of that of, preferably substantially the same as, the $IC_{50}$ of the corresponding reference molecule when assayed as described above.

For example, the assay used may be an assay of competitive inhibition of binding of MCP-1 by membrane bound MCP-1 receptor (CCR2B) and the MCP-1 binding molecules of the invention, e.g. using SPA technology as hereinafter described in the Examples.

Most preferably, the human MCP-1 antibody comprises at least

a) one heavy chain which comprises a variable domain having an amino acid sequence substantially identical to that shown in Seq. Id. No. 1 starting with the amino acid at position 1 and ending with the amino acid at position 122 and the constant part of a human heavy chain; and

b) one light chain which comprises a variable domain having an amino acid sequence substantially identical to that shown in Seq. Id. No. 2 starting with the amino acid at position 1 and ending with the amino acid at position 109 and the constant part of a human light chain.

The constant part of a human heavy chain may be of the $\gamma_1$, $\gamma_2$, $\gamma_3$, $\gamma_4$, $\mu$, $\alpha_1$, $\alpha_2$, $\delta$ or $\varepsilon$ type, preferably of the $\gamma$ type, more preferably of the $\gamma_4$ type, whereas the constant part of a human light chain may be of the $\kappa$ or $\lambda$ type (which includes the $\lambda_1$, $\lambda_2$ and $\lambda_3$ subtypes) but is preferably of the $\kappa$ type. The amino acid sequences of all these constant parts are given in Kabat et al ibid.

An MCP-1 binding molecule of the invention may be produced by recombinant DNA techniques. In view of this, one or more DNA molecules encoding the binding molecule must be constructed, placed under appropriate control sequences and transferred into a suitable host organism for expression.

In a very general manner, there are accordingly provided

(i) DNA molecules encoding a single domain MCP-1 binding molecule, of the invention, a single chain MCP-1 binding molecule of the invention, a heavy or light chain or fragments thereof or a MCP-1 binding molecule of the invention and

(ii) the use of the DNA molecules of the invention for the production of a MCP-1 binding molecule of the invention by recombinant means.

The present state of the art is such that the skilled worker in the art is able to synthesize the DNA molecules of the invention given the information provided herein i.e. the amino acid sequences of the hypervariable regions and the DNA sequences coding for them. A method for constructing a variable domain gene is for example described in EPA 239 400 and may be briefly summarized as follows: A gene encoding a variable domain of a MAb of whatever specificity is cloned. The DNA segments encoding the framework and hypervariable regions are determined and the DNA segments encoding the hypervariable regions are removed so that the DNA segments encoding the framework regions are fused together with suitable restriction sites at the junctions. The restriction sites may be generated at the appropriate positions by mutagenesis of the DNA molecule by standard procedures. Double stranded synthetic CDR cassettes are prepared by DNA synthesis according to the sequences given in Seq. Id. No. 1 or 2. These cassettes are provided with sticky ends so that they can be ligated at the junctions of the framework

Furthermore, it is not necessary to have access to the mRNA from a producing hybridoma cell line in order to obtain a DNA construct coding for the MCP-1 binding molecules of the invention. Thus PCT application WO 90/07861 gives full instructions for the production of an antibody by recombinant DNA techniques given only written information as to the nucleotide sequence of the gene. The method comprises the synthesis of a number of oligonucleotides, their amplification by the PCR method, and their splicing to give the desired DNA sequence.

Expression vectors comprising a suitable promoter or genes encoding heavy and light chain constant parts are publicly available. Thus, once a DNA molecule of the invention is prepared it may be conveniently transferred in an appropriate expression vector. DNA molecules encoding single chain antibodies may also be prepared by standard methods, for example, as described in WO 88/1649.

In view of the foregoing no hybridoma or cell line deposit is necessary to comply with the criteria of sufficiency of description.

In a particular embodiment the invention includes first and second DNA constructs for the production of an MCP-1 binding molecule as described below:

The first DNA construct encodes a heavy chain or fragment thereof and comprises

a) a first part which encodes a variable domain comprising alternatively framework and hypervariable regions, said hypervariable regions being in sequence CDR1, CDR2 and CDR3 the amino acid sequences of which are shown in Seq. Id. No. 1; this first part starting with a codon encoding the first amino acid of the variable domain and ending with a codon encoding the last amino acid of the variable domain, and

b) a second part encoding a heavy chain constant part or fragment thereof which starts with a codon encoding the first amino acid of the constant part of the heavy chain and ends with a codon encoding the last amino acid of the constant part or fragment thereof, followed by a stop codon.

Preferably, this first part encodes a variable domain having an amino acid sequence substantially identical to the amino acid sequence as shown in Seq. Id. No. 1 starting with the amino acid at position 1 and ending with the amino acid at position 122. More preferably the first part has the nucleotide sequence as shown in Seq. Id. No. 1 starting with the nucleotide at position 1 and ending with the nucleotide at position 366. Also preferably, the second part encodes the constant part of a human heavy chain, more preferably the constant part of the human γ4 chain. This second part may be a DNA fragment of genomic origin (comprising introns) or a cDNA fragment (without introns).

The second DNA construct encodes a light chain or fragment thereof and comprises

a) a first part which encodes a variable domain comprising alternatively framework and hypervariable regions; said hypervariable regions being CDR1', CDR2' and CDR3', the amino acid sequences of which are shown in Seq. Id. No. 2; this first part starting with a codon encoding the first amino acid of the variable domain and ending with a codon encoding the last amino acid of the variable domain, and

b) a second part encoding a light chain constant part or fragment thereof which starts with a codon encoding the first amino acid of the constant part of the light chain and ends with a codon encoding the last amino acid of the constant part or fragment thereof followed by a stop codon.

Preferably, this first part encodes a variable domain having an amino acid sequence substantially identical to the amino acid sequence as shown in Seq. Id. No. 2 starting with the amino acid at position 1 and ending with the amino acid at position 109. More preferably, the first part has the nucleotide sequence as shown in Seq. Id. No. 2 starting with the nucleotide at position 1 and ending with the nucleotide at position 327. Also preferably the second part encodes the constant part of a human light chain, more preferably the constant part of the human κ chain.

The invention also includes MCP-1 binding molecules in which one or more, typically only a few, of the residues of CDR1, CDR2, CDR3, CDR1', CDR2' or CDR3' are changed from the residues shown in Seq Id No. 1 and Seq. Id. No. 2; for instance by mutation e.g. site directed mutagenesis of the corresponding DNA sequences. The invention includes the DNA sequences coding for such changed MCP-1 binding molecules. The invention also includes binding molecules in which one or more, typically only a few, of the residues of the framework regions are changed from the residues shown in Seq Id No. 1 and Seq. Id No. 2.

In the first and second DNA constructs, the first and second parts may be separated by an intron, and, an enhancer may be conveniently located in the intron between the first and second parts. The presence of such an enhancer which is transcribed but not translated, may assist in efficient transcription. In particular embodiments the first and second DNA constructs comprise the enhancer of a heavy chain gene advantageously of human origin.

Each of the DNA constructs are placed under the control of suitable expression control sequences, in particular under the control of a suitable promoter. Any kind of promoter may be used, provided that it is adapted to the host organism in which the DNA constructs will be transferred for expression. However, if expression is to take place in a mammalian cell, it is particularly preferred to use the promoter of an immunoglobulin gene.

The desired antibody may be produced in a cell culture or in a transgenic animal. A suitable transgenic animal may be obtained according to standard methods which include micro injecting into eggs the first and second DNA constructs placed under suitable control sequences transferring the so prepared eggs into appropriate pseudo-pregnant females and selecting a descendant expressing the desired antibody.

When the antibody chains are produced in a cell culture, the DNA constructs must first be inserted into either a single expression vector or into two separate but compatible expression vectors, the latter possibility being preferred.

Accordingly, the invention also provides an expression vector able to replicate in a prokaryotic or eukaryotic cell line which comprises at least one of the DNA constructs described above.

Each expression vector containing a DNA construct is then transferred into a suitable host organism. When the DNA constructs are separately inserted on two expression vectors, they may be transferred separately, i.e. one type of vector per cell, or co-transferred, this latter possibility being preferred. A suitable host organism may be a bacterium, a yeast or a mammalian cell line, this latter being preferred. More preferably, the mammalian cell line is of lymphoid origin, e.g. a myeloma, hybridoma or a normal immortalised B-cell, which conveniently does not express any endogenous antibody heavy or light chain, e.g. the SP 2/0 cell line.

For expression in mammalian cells it is preferred that the MCP-1 binding molecule coding sequence is integrated into the host cell DNA within a locus which permits or favours high level expression of the MCP-1 binding molecule. Cells in which the MCP-1 binding molecule coding sequence is integrated into such favourable loci may be identified and selected on the basis of the levels of the MCP-1 binding molecule which they express. Any suitable selectable marker may be used for preparation of host cells containing the MCP-1 binding molecule coding sequence; for instance, a dhfr gene/methotrexate or equivalent selection system may be used. Systems for expression of the MCP-1 binding molecules of the invention include GS-based amplification/selection systems, such as those described in EP 0256055 B, EP 0323997 B and EP 0338841 B.

In a further aspect of the invention there is provided a process for the production of an MCP-1 binding molecule with comprises (i) culturing an organism which is co-transformed with expression vectors as defined above and (ii) recovering the MCP-1 binding molecule from the culture.

Most preferably the MCP-1 binding molecule of the invention is a human antibody, e.g. the AAV293, AAV294 or ABN912 antibodies, and may be produced by cultivation of a corresponding hybridoma cell line, or preferably from a recombinant cell line containing DNA coding for the human antibody, including DNA altered to alter antibody isotype or other antibody function or property.

In accordance with the present invention it has been found that the AAV294 and more especially the AAV293 and ABN912 antibodies cross-react with recombinant human eotaxin-1. As such these antibodies advantageously interact with eotaxin-1 as well as MCP-1, and may be used to inhibit binding of eotaxin-1 to its receptor, in addition to inhibiting binding of MCP-1 to its receptor. Eotaxin secretion is implicated in allergic diseases and disorders including allergic inflammatory airways diseases, such as asthma. Antibodies, in particular chimeric and CDR-grafted antibodies and especially human antibodies which have binding specificity for both MCP-1 and eotaxin, e.g. human MCP-1 and human eotaxin, and the use of such antibodies for the treatment of diseases mediated by MCP-1 or eotaxin, are hereby described.

Thus in a further aspect the invention includes an antibody to MCP-1 which cross-reacts with eotaxin.

Advantageously the antibodies of this aspect of the invention are antibodies which are capable of inhibiting the binding of MCP-1 to its receptor and capable of inhibiting the binding of eotaxin to its receptor.

In yet further aspects the invention includes:

> i) use of an antibody to MCP-1 which cross-reacts with eotaxin-1 which is capable of inhibiting the binding of MCP-1 and eotaxin-1 to their receptors, for the preparation of a medicament for the treatment of an MCP-1- or eotaxin-1-mediated disease or disorder;
> ii) a pharmaceutical composition comprising an antibody to MCP-1 which cross-reacts with eotaxin-and which is capable of inhibiting the binding of MCP-1 and eotaxin to their receptors, in combination with a pharmaceutically acceptable excipient, diluent or carrier.

In these further aspects the MCP-1 antibody preferably cross-reacts with eotaxin-1 and the eotaxin-mediated diseases are preferably eotaxin-1-mediated diseases.

For the purposes of the present description an antibody is "capable of inhibiting the binding of MCP-1 and eotaxin to their receptors" if the antibody is capable of inhibiting the binding of MCP-1 and eotaxin to their receptors substantially to the same extent as the AAV294, AAV293 or ABN912 antibody, wherein "to the same extent" has meaning as defined above.

In the present description the phrase "MCP-1 mediated disease" and "eotaxin-mediated disease" encompasses all diseases and medical conditions in which MCP-1 or eotaxin, in particular eotaxin-1, plays a role, whether directly or indirectly, in the disease or medical condition, including the causation, development, progression, persistence or pathology of the disease or condition.

In the present description the terms "treatment" or "treat" refer to both prophylactic or preventative treatment as well as curative or disease modifying treatment, including treatment of patient at risk of contracting the disease or suspected to have contracted the disease as well as patients who are ill or have been diagnosed as suffering from a disease or medical condition, and includes suppression of clinical relapse.

The AAV293 and ABN912 antibodies have binding affinity for MCP-1 which is higher than affinities previously reported for anti-MCP-1 antibodies, e.g. anti human MCP-1 antibodies. Thus ABN912 has a dissociation equilibrium constant $K_D$ for binding to MCP-1 of less than about 50 pM, e.g. about 43pM. This high binding affinity makes the ABN912 particularly suitable for therapeutic applications.

Thus in a yet further aspect the invention provides an antibody to MCP-1 which has a $K_D$ for binding to MCP-1 of about 50 pM or less. This aspect of the invention also includes uses methods and compositions for such high affinity antibodies, as described above for antibodies to MCP-1 that cross-react with eotaxin.

Furthermore in accordance with the present invention it has been found that the ABN912 antibody binds to an antigenic epitope of MCP-1 which includes the Arginine residue at position 24 of MCP-1. Thus advantageously the ABN912 antibody is able to interfere directly with binding of MCP-1 to its receptor (CCR2B); Arg24 is an important residue of MCP-1 for binding of MCP-1 to CCR2B. In addition the ABN912 binding site includes the Arg18, and Lys49 residues of MCP-1.

Accordingly in a yet further aspect the invention includes an antibody to MCP-1 which binds to an antigenic epitope of MCP-1 which includes the Arginine residue at position 24 of MCP-1. Preferably the antigenic epitope also includes the Arginine residue at position 18 and the Lysine residue at position 49 of MCP-1. Similarly this aspect of the invention includes uses, methods and compositions as described above for antibodies to MCP-1 which cross-react with eotaxin.

MCP-1 binding molecules as defined above, in particular MCP-1 binding molecules according to the first and second aspects of the invention; antibodies to MCP-1 which cross-react with eotaxin, in particular antibodies which are capable of inhibiting the binding of MCP-1 and eotaxin to their receptors; antibodies to MCP-1 which have a $K_D$ for binding to MCP-1 of about 50 pM or less; and antibodies to MCP-1 which binds to an antigenic epitope of MCP-1 which includes the Arginine residue at position 24 of MCP-1 are hereinafter referred to as "Antibodies of the Invention".

Preferably the Antibodies of the Invention are MCP-1 binding molecules according to the first and second aspect of the invention. Advantageously the Antibodies of the Invention are human antibodies, most preferably the ABN912 antibody or direct equivalent thereof.

The Antibodies of the invention inhibit the effects of MCP-1 on its target cells and thus are indicated for use in the treatment of MCP-1 mediated diseases and disorders. These and other pharmacological activities of the Antibodies of the Invention may be demonstrated in standard test methods, for example as described below:

1. Inhibition of MCP-1 binding to CCR2B expressing cells

A prerequisite for signalling and effector functions of MCP-1 is its interaction with the receptor CCR2B. Scintillation proximity assay (SPA) technology is used to demonstrate that Antibodies of the Invention inhibit MCP-1 binding to cell membranes which express this receptor.

Membranes of CCR2B expressing CHO cells are incubated with a range of concentration as of target antibody (e.g. $10^{-14}$ M to $10^{-8}$M) and the residual binding of (125-1)-MCP-1 is measured by SPA using wheat germ agglutinin beads. Antibodies of the Invention typically have an $IC_{50}$s in the range from about 0.1 to about 10nM, especially of about 0.5nM (e.g. 461±206pM) when tested in this assay.

2. Inhibition of MCP-1 mediated signalling

The potential of Antibodies of the Invention to inhibit physiological effects elicited by MCP-1 is determined by measuring the MCP-1 induced intracellular $Ca^{2+}$ mobilisation in the presence and absence of the antibody.

Measurement of calcium response is performed with a stably transacted CCR2B expressing CHO cell line and with THP-1 cells by use of fluorescent dyes and FACS analysis as herein after described in the Examples. Antibodies of the Invention typically have an $IC_{50}$s in the range from about 0.05 to about 10nM, especially of about 0.5nM (e.g. 390±20pM) when tested in this assay.

The Antibodies of the Invention advantageously cross-react with eotaxin, in particular eotaxin-1, and thus advantageously may inhibit the effects of eotaxin on its target cells and thus are additionally indicated for use in the treatment of eotaxin may be determined using optical biosensor technology, such as BIAcore (Karlsson et al. J. Immunol.

Meth. 1991; 145:229-240).

As indicated in the above assays Antibodies of the Invention potently block the effects of MCP-1, and preferably cross-react with eotaxin. Accordingly, the Antibodies of the Invention have pharmaceutical utility as follows:

Antibodies of the Invention are useful for the prophylaxis and treatment of MCP-1 or eotaxin mediated diseases or medical conditions. MCP-1 plays an important role in leukocyte trafficking, in particular in monocyte migration of inflammatory sites and thus the Antibodies of the Invention may be used to inhibit monocyte migration e.g. in the treatment of inflammatory conditions, allergies an allergic conditions, autoimmune diseases, graft rejection, cancers which involve leukocyte infiltration, stenosis or restenosis, atherosclerosis, rheumatoid arthritis and osteoarthritis.

Diseases or conditions which may be treated with the Antibodies of the Invention include: Inflammatory or allergic conditions, including respiratory allergic diseases such as asthma, allergic rhinitis, COPD, hypersensitivity lung diseases, hypersensitivity pneumonitis, interstitial lung disease (ILD), (e.g. idiopathic pulmonary fibrosis, or ILD associated with autoimmune diseases such as RA, SLE, etc.); anaphylaxis or hypersensitivity responses, drug allergies (e.g. to penicillins or cephalosporins), and insect sting allergies; inflammatory bowel diseases, such as Crohn's disease and ulcerative colitis; spondyloarthropathies, sclerodoma; psoriasis and inflammatory dermatoses such as dermatitis, eczema, atopic dermatitis, allergic contact dermatitis, urticaria; vasculitis;

Autoimmune diseases, in particular autoimmune diseases with an aetiology including an inflammatory component such as arthritis (for example rheumatoid arthritis, arthritis chronica progrediente, psoriatic arthritis and arthritis deformans) and rheumatic diseases, including inflammatory conditions and rheumatic diseases involving bone loss, inflammatory pain, hypersensitivity (including both airways hypersensitivity and dermal hypersensitivity) and allergies. Specific auto-immune diseases for which Antibodies of the Invention may be employed include autoimmune haematological disorders (including e.g. hemolytic anaemia, aplastic anaemia, pure red cell anaemia and idiopathic thrombocytopenia), systemic lupus erythematosus, polychondritis, sclerodoma, Wegener granulomatosis, dermatomyositis, chronic active hepatitis, myasthenia gravis, psoriasis, Steven-Johnson syndrome, idiopathic sprue, autoimmune inflammatory bowel disease (including e.g. ulcerative colitis, Crohn's disease and Irritable Bowel Syndrome), autoimmune thyroiditis, Behcet's disease, endocrine ophthalmopathy, Graves disease, sarcoidosis, multiple sclerosis, primary biliary cirrhosis, juvenile diabetes (diabetes mellitus type I), uveitis (anterior and posterior), keratoconjunctivitis sicca and vernal keratoconjunctivitis, interstitial lung fibrosis, and glomerulonephritis (with and without nephrotic syndrome, e.g. including idiopathic nephrotic syndrome or minimal change nephropathy);

graft rejection (e.g. in transplantation including heart, lung, combined heart-lung, liver, kidney, pancreatic, skin, or corneal transplants) including allograft rejection or xenograft rejection or graft-versus-host disease, and organ transplant associated arteriosclerosis;

atherosclerosis;

cancer with leukocyte infiltration of the skin or organs;

stenosis or restenosis of the vasculature, particularly of the arteries, e.g. the coronary artery, including stenosis or restenosis which results from vascular intervention, as well as neointimal hyperplasia;

and other diseases or conditions involving inflammatory responses including reperfusion injury, hematologic malignancies, cytokine induced toxicity (e.g. septic shock or endotoxic shock), polymyositis, dermatomyositis, and granulomatous diseases including sarcoidosis.

Antibodies of the Invention are particularly useful for treating diseases of bone and cartilage metabolism including osteoarthritis, osteoporosis and other inflammatory arthritides, e.g. rheumatoid arthritis, and bone loss in general, including age-related bone loss, and in particular periodontal disease.

For the above indications, the appropriate dosage will, of course, vary depending upon, for example, the particular Antibody of the Invention to be employed, the host, the mode of administration and the nature and severity of the condition being treated. However, in prophylactic use, satisfactory results are generally indicated to be obtained at dosages from about 0.05 mg to about 10 mg per kilogram body weight, more usually from about 0.1 mg to about 5 mg per kilogram body weight. The frequency of dosing for prophylactic use will normally be in the range from about once per week up to about once every 3 months, more usually in the range from about once every 2 weeks up to about once every 10 weeks, e.g. once every 4 or 8 weeks. Antibody of the Invention is conveniently administered parenterally, intravenously, e.g. into the antecubital or other peripheral vein, intramuscularly, or subcutaneously. For example, a prophylactic treatment typically comprises administering the Antibody of the Invention once per month to once every 2 to 3 months, or less frequently.

Pharmaceutical compositions of the invention may be manufactured in conventional manner. A composition according to the invention is preferably provided in lyophilized form. For immediate administration it is dissolved in a suitable aqueous carrier, for example sterile water for injection or sterile buffered physiological saline. If it is considered desirable to make up a solution of larger volume for administration by infusion rather as a bolus injection, it is advantageous to incorporate human serum albumin or the patient's own heparinised blood into the saline at the time of formulation.

The presence of an excess of such physiologically inert protein prevents loss of antibody by adsorption onto the walls of the container and tubing used with the infusion solution. If albumin is used, a suitable concentration is from 0.5 to 4.5% by weight of the saline solution.

The invention is further described by way of illustration only in the following Examples, which refer to the accompanying diagrams:

Figure 1

A - a graph showing eosinophilperoxidase (EPO) activity for various punch biopsies from treated and untreated rhesus monkeys;
B - a similar graph showing myeloperoxidase (MPO) activity for the biopsies;

Figure 2 - photographs showing eosinophil staining for histology samples from four rhesus monkeys both before and after treatment with ABN912 and CGP44290 (a control antibody);
Figure 3 - graphs showing Th cell migration (%) in human skin transplants for various treatment regimes, and

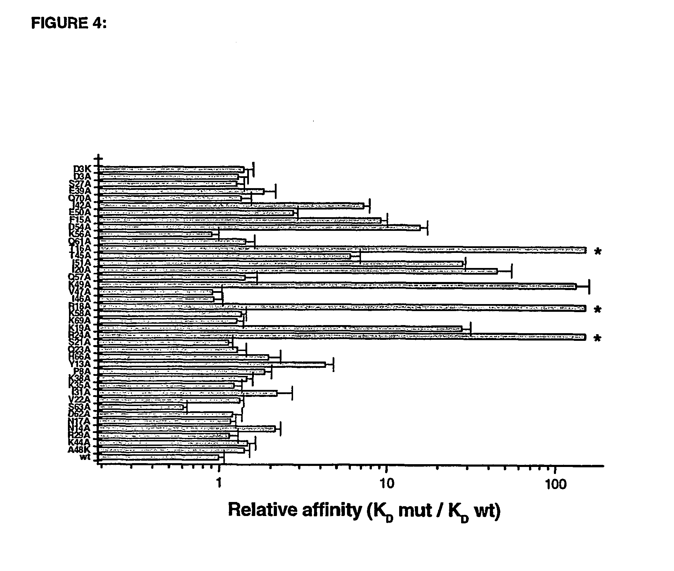

Figure 4 - a graph showing the relative binding affinities of ABN912 mutants for binding to MCP-1.

### EXAMPLES

Transgenic mice engineered to express the human IgG/κ repertoire instead of the murine immunoglobulin repertoire (Fishwild et al., 1996, Nat Biotechnol., 14, 845-851) are used to generate antibodies to human MCP-1. B cells from these mice are immortalized by standard hybridoma technology and murine hybridoma cells are obtained which secrete the human IgG3/κ antibody AAV293.

### Example 1: Immunisation of Mice and Generation of the Hybridoma Cell Line

Immunisation

Four Medarex mice (mice Nos. 16194-16197, Medarex Inc. Annadale, NJ, USA) are immunised with recombinant human MCP-1 (R&D Systems, Minneapolis, MN, USA), 100μg protein per mouse in Complete Freund's Adjuvant on days 0 and 14 (i.p.) and day 26 (i.v.). At day 41 none of the mice shows detectable serum antibody. The mice are further immunised with rhMCP-1, 100μg protein per mouse s.c. in Incomplete Freund's Adjuvant on days 49 and 65. When assayed on day 106, a substantial anti-MCP-1 antibody titer is detected in the serum of one of the mice (mouse No. 16194). This mouse is boosted 7 additional times before fusion: 100μg protein per mouse in saline on days 106 (i.p.), 119 (s.c.) and 135 (i.p.), and 25μg protein per mouse in saline on days -4 (x2 - i.v. and i.p.) and -3 and -2 (both i.p.) before fusion.

Fusion and Hybridoma Selection

On the day of the fusion mouse 16194 is killed by $CO_2$ inhalation and total spleen cells ($4.8 \times 10^7$) are fused by a routine method using PEG 4000 with PAI-O cells ($5 \times 10^7$), a mouse myeloma cell line. Fused cells are plated out in 720 wells (1ml/well) containing a feeder layer of mouse peritoneal cells (Balb/c mice), in HAT supplemented RPMI 1640, 10% heat inactivated fetal calf serum, $5 \times 10^{-5}$ M β-mercaptoethanol 50μg/ml Gentamicin. Culture medium is exchanged every 4th day and after 14 days HAT medium is exchanged for HT medium, i.e. Aminopterin is omitted. Of the initial 720 wells plated, 461 wells (64%) are positive for hybridoma growth. Supernatants are collected and screened for MCP-1 reactive monoclonal antibodies in an ELISA. Seven monoclonal antibodies of the IgG subclass are identified. Cloning is done using 4 x 96 well microtiter plates, plating 0.5 cells/100μl per well. Clones are checked microscopically after 8 days, 100μl of growth medium is added and supernatant tested the following day in an ELISA. The most reactive hybridoma, clone 149 is selected for further cloning and characterisation. Hybridoma subclone 149-12 is selected based on the inhibitory activity in a rhMCP-1 dependent calcium mobilisation assay of its IgG3/κ monoclonal antibody product, AAV293.

**Purity and partial amino acid sequences of heavy and light chain Amino acid sequencing**

Light and heavy chains of the purified antibody AAV293 are separated by SDS-PAGE and the amino-terminal amino acids determined by Edman degradation. cDNA sequences coding for the heavy and light chain variable domains are obtained by PCR amplification of cDNA obtained from mRNA from the cloned hybridoma cells and fully sequenced. The amino-terminal amino acid sequences of heavy and light chain variable domains and the corresponding DNA sequences are given in Seq. Id no. 1 and Seq Id No. 2 below, in which the CDRs are shown in bold type.


## Seq. Id no. 1

```
                                           30                                     60
GAG GTG CAG CTG GTG CAG TCT GGG GGA GGC TTG GTC CAG CCT GGG GGG TCC CTG AGA CTC
Glu Val Gln Leu Val Gln Ser Gly Gly Gly Leu Val Gln Pro Gly Gly Ser Leu Arg Leu
                                           10                                     20

                                           90        CDR1                         120
TCC TGT GCA GCC TCT GGA TTC ACC TTT AGT CAC TAC TGG ATG AGC TGG GTC CGC CAG GCT
Ser Cys Ala Ala Ser Gly Phe Thr Phe Ser His Tyr Trp Met Ser Trp Val Arg Gln Ala
                                           30                                     40

                                           150          CDR2                      180
CCA GGG AAA GGG CTG GAG TGG CTG GCC AAC ATA GAG CAA GAT GGA AGT GAG AAA TAC TAT
Pro Gly Lys Gly Leu Glu Trp Val Ala Asn Ile Glu Gln Asp Gly Ser Glu Lys Tyr Tyr
                                           50                                     60

                                           210                                    240
GTG GAC TCT GTG AAG GGC CGA TTC ACC ATC TCC AGA GAC AAC GCC AAG AAT TCA CTG TAT
Val Asp Ser Val Lys Gly Arg Phe Thr Ile Ser Arg Asp Asn Ala Lys Asn Ser Leu Tyr
                                           70                                     80

                                           270                                    300
CTG CAA ATG AAC AGT CTG AGA GCC GAG GAC ACG GCT GTG TAT TTC TGT GCG AGG GAT CTT
Leu Gln Met Asn Ser Leu Arg Ala Glu Asp Thr Ala Val Tyr Phe Cys Ala Arg Asp Leu
                                           90                                     100

              CDR3                         330                                    360
GAA GGT CTA CAT GGG GAT GGG TAC TTC GAT CTC TGG GGC CGT GGC ACC CTG GTC ACC GTC
Glu Gly Leu His Gly Asp Gly Tyr Phe Asp Leu Trp Gly Arg Gly Thr Leu Val Thr Val
```

```
                                           110                                      120
  TCT  TCA
  Ser  Ser
```

## Seq. Id no. 2

```
                                            30                                       60
  GCC ATC CAG TTG ACA CAG TCT CCA TCC TCC CTG TCT GCA TCT GTA GGA GAC AGA GTC ATC
  Ala Ile Gln Leu Thr Gln Ser Pro Ser Ser Leu Ser Ala Ser Val Gly Asp Arg Val Ile
                                            10                                       20


                          CDR1             90                                      120
  CTC ATC TGC CGG GCA AGT CAG GGC GTT AGC AGT GCT TTA GCC TGG TAT CAG CAG AAA CCA
  Leu Thr Cys Arg Ala Ser Gln Gly Val Ser Ser Ala Leu Ala Trp Tyr Gln Gln Lys Pro
                                            30                                       40



                                           150   CDR2                              180
  GGG AAA GCT CCT AAG CTC CTG ATC TAT GAT GCC TCC AGT TTG GAA AGT GGG GTC CCA TCA
  Gly Lys Ala Pro Lys Leu Leu Ile Tyr Asp Ala Ser Ser Leu Glu Ser Gly Val Pro Ser
                                            50                                       60


                                           210                                      240
  AGG TTC AGC GGC AGT GGA TCT GGG CCA GAT TTC ACT CTC ACC ATC AGC AGC CTG CAG CCT
  Arg Phe Ser Gly Ser Gly Ser Gly Pro Asp Phe Thr Leu Thr Ile Ser Ser Leu Gln Pro
                                            70                                       80


                                           270   CDR3                              300
  GAA GAT TTT GCA ACT TAT TTC TGT CAA CAG TTT AAT AGT TAC CCT CTC ACT TTC GGC GGA
  Glu Asp Phe Ala Thr Tyr Phe Cys Gln Gln Phe Asn Ser Tyr Pro Leu Thr Phe Gly Gly
                                            90                                      100



  GGG ACC AAG GTG GAA ATC AAA CGA ACT
  Gly Thr Lys Val Glu Ile Lys Arg Thr
```

A further anti-MCP-1 monoclonal antibody is also obtained as described above, the IgG1/κ monoclonal antibody product, AAV294. This antibody binds MCP-1 with approximately 3-fold less affinity than the AAV293 antibody and is found to have $V_H$ and $V_L$ amino acid sequences which are identical to those of the AAV293 antibody except that, in $V_H$ AAV294 has Val in place of Ala at position 24, Phe in place of Tyr at position 60 and Ser in place of Asn at position 74, and in $V_L$ AAV294 has Tyr in place of Ser at position 30 and Thr in place of Pro in position 69.

**Construction of expression vectors for heavy and light chain**

The cloned $V_L$ and $V_H$ encoding sequences were amplified by PCR and inserted via appropriate restriction sites into cassette vectors providing the immunoglobulin promoter, the leader sequences from the RFT2 antibody (Heinrich et al. (1989) J. Immunol. 143, 3589-97), part of the J-segments and a splice donor site. The light chain cassette containing the entire $V_L$ region, promoter and leader sequence for secretion was transferred into an expression vector containing the human Ck gene, the immunoglobulin heavy chain enhancer, and the modified murine dhfr cDNA for selection by methotrexate (MTX).

The heavy chain cassette was transferred accordingly into an expression vector encoding the human IgG4 gene, the immunoglobulin heavy chain enhancer, and the neomycin resistance gene for selection.

Both heavy and light chain are in a configuration in the expression vectors that resembles the genomic configuration of rearranged immunoglobulin genes which is thought to be crucial for high level expression.

For antibody production the above vectors are co-transfected into an appropriate host cell line, e.g. the SP2/0 cell line. Cells containing the vector sequences are selected by methotrexate selection, and selected cell lines are cultured to express the ABN912 antibody (human IgG4/κ anti-human MCP-1 antibody).

Expression vectors carrying the heavy and light chain genes of NVP-ABN912, respectively, were linearized and Sp2/0 cells were transfected by electroporation. The transfected cells were grown for 20 hours in non-selective RPMI medium with foetal calf serum (FCS) and G418 selection was applied for 48 -72 hours at 1.4 mg/ml. Transfected pools were adapted to FCS free RPMI medium containing commonly used additives (Pyruvate, glutamine, human serum albumin, transferrin, insulin). High producer clones were isolated after two step amplification with methotrexate at 200nM and 1 μM. Production stability of the clones and sub-clones was assessed in T175 cultures over a period of four-five months of continuous cultivation, with additional spinner experiments. For high producer clones a scale-up from lab-scale to bioreactor cultivation was performed. Several high producer cell lines, useful for production of ABN912, were obtained. The maximum amount of accumulated product obtained in overgrowth cultures was 504 mg/l.

### Example 2: Biochemical and Biological Data

The human monoclonal antibody ABN912 binds to human MCP-1 and neutralises its function in vitro. The monoclonal antibody is further characterized for its binding to recombinant human MCP-1 by two independent biochemical methods, by Biacore analysis and Scintilation proximity assay (SPA) analysis. The specificity of the ABN912 antibody for other CC-chemokines or non-human MCP-1 is assessed by BIAcore and the inhibition of binding of MCP-1 to cells expressing CCR2B by ABN912 is demonstrated by SPA. The biological activity of ABN912 towards recombinant and naturally produced MCP-1 is demonstrated in $Ca^{2+}$ mobilisation assays in cells expressing CCR2B. The activity of ABN912 towards its natural target cells, human peripheral blood monocytes (hPBMC), is demonstrated by a MCP-1 induced chemotaxis assay.

### 2.1 BIAcore analysis

The association and dissociation rate constants for the binding of recombinant human MCP-1 to immobilised ABN912 are determined by BIAcore analysis and the $K_D$ value derived. ABN912 is immobilized on a sensorchip surface and binding of recombinant MCP-1 measured by surface plasmon resonance (BIACORE 2000 Instrument Handbook, March 1999 (Version AC); http:www.biacore.com). The results obtained are given in the Table below.

| | | |
|---|---|---|
| Association rate constant [$M^{-1}s^{-1}$] | (n=30) | $(1.3 \pm 0.1) \times 10^7$ |
| Dissociation rate constant [$s^{-1}$] | (n=30) | $(5.0 \pm 0.1) \times 10^{-4}$ |
| Dissociation equilibrium constant $K_D$ [M] | (n=30) | $(43.0 \pm 2.9) \times 10^{-12}$ |

ABN912 binds to recombinant human MCP-1 with a very high affinity.

### 2.2. Chemokine selectivity and species specificity profile

In order to determine the specificity of the MCP-1 interaction with ABN912, a number of non-human MCP-1 and CC-chemokines are assessed for their potential to interact with ABN912 by BIAcore.

### 2.2.1 Interactions with non-primate MCP-1

A series of MCP-1s from species commonly used in laboratories is assessed for binding to the ABN912 antibody. The measurements are carried out by injecting a 5 nM chemokine solution into the BIAcore flowcell that was previously loaded with ABN912. After 8 minutes the amount of bound chemokine is measured for each chemokine investigated. The results obtained are shown in the Table below as the percent binding of each chemokine compared to recombinant human MCP-1 (100%).

| Chemokine | Percent binding of ABN912 Average $\pm$ SEM, n = 3 |
|---|---|
| Rec hu MCP-1 | 100 |

(continued)

| Chemokine | Percent binding of ABN912 Average $\pm$ SEM, n = 3 |
|---|---|
| Rec mouse JE/MCP-1 | -0.4 $\pm$ 0.2 |
| Rec rat MCP-1 | -0.2 $\pm$ 2.3 |
| Rec rabbit MCP-1 | 0.9 $\pm$ 0.4 |
| Rec dog MCP-1 | 1.3 $\pm$ 0.3 |
| Rec pig MCP-1 | 0.5 $\pm$ 0.4 |
| Rec guinea pig MCP-1 | 2.3 $\pm$ 0.2 |
| ABN912 is specific for human MCP-1 and does not cross-react with the MCP-1 of any other species tested. | |

### 2.2.2 Interactions with recombinant chemokines

In order to determine the cross-reactivity profile of ABN912 to other CC-chemokines, their binding potential to the antibody is assessed by the procedure described above. Percent binding of each chemokine compared to recombinant human MCP-1 is shown in the Table below.

| Chemokine | Binding to ABN912 Average $\pm$ SEM, n = 3 |
|---|---|
| rh MCP-1 | 100 |
| rh MCP-2 | 6.2 $\pm$ 0.9 |
| rh MCP-3 | 4.3 $\pm$ 4.3 |
| rh MCP-4 | -2.4 $\pm$ 0.6 |
| rh LEC | -2.8 $\pm$ 0.3 |
| rh RANTES | -2.7 $\pm$ 0.4 |
| rh MIP- 1$\alpha$ | -2.8 $\pm$ 0.9 |
| rh MIP-1$\beta$ | -2.9 $\pm$ 0.1 |
| rh eotaxin | 52.6 $\pm$ 1.9 |
| ABN912 is specific for human MCP-1 and does not cross-react with MCP-2, MCP-3, MCP-4, LEC, RANTES, MIP-1$\alpha$ and MIP-1$\beta$, but does significantly cross-react with human eotaxin, i.e. human eotaxin-1. The AAV294 is also found to cross-react significantly with eotaxin. | |

### 2.3 Inhibition of MCP-1 binding to CCR2B expressing cells

SPA (Scintillation Proximity Assay) techology is used to show that the ABN912 antibody prevents MCP-1 from binding to cell membranes expressing the CCR2B receptor. Membranes of CCR2B expressing CHO cells (CHO#84 - see below) are incubated with a range of concentrations of ABN912 ($10^{-14}$M to $10^{-8}$M) and the residual binding of radioactive (125-I)-MCP-1 is measured by SPA using wheat germ agglutinin beads. The average $IC_{50}$ [M] obtained from three independent experiments is (461 $\pm$ 206) x $10^{-12}$. ABN912 prevents MCP-1 from binding to cell membranes expressing the CCR2B receptor.

**2.4 Inhibition of MCP-1 mediated signalling**

An early event of MCP-1 induced signalling is the mobilisation of intracellular $Ca^{2+}$ which can be measured by the use of fluorescent dyes.

Measurement of calcium responses are performed using cell line CHO#84, a CHO cell line stably transfected to express chemokine receptor CCR2B. The CHO#84 cells are cultured in MEM alpha medium without ribonucleases and deoxyribonucleases, with glutamax-1 supplemented with 10% dialysed FCS, 200U/ml Penicillin/Streptomycin and 80nM methotrexate as selective agent. When cell culture is dense but before confluence, cells are washed with PBS and detached by a short incubation with trypsin-EDTA (1 min max).

The CHO#84 cells are washed once in RPMI, by 7 min centrifugation at 250g and resuspended at $3.5 \times 10^6$ cells/ml in Hepes buffer 0.5% BSA containing 0.04% pluronic acid, $1.0\mu$ M fura red and $0.3\mu$M fluo-3. The cells are loaded with these fluorescent calcium probes for 1 hour at room temperature in the dark, with gentle agitation from time to time (6-8 times). Then the cells are harvested twice in Hepes buffer 0.5% BSA by centrifugation, and the pellet is resuspended at 1.5 to $2 \times 10^6$ cells/ml in Hepes buffer 0.5% BSA. The cells are now ready for stimulation and calcium measurement and are stored at room temperature in the dark until use.

Both antibodies and chemokine (MCP-1, R&D Systems, Minneapolis, MN, USA) are prepared as twenty-fold concentration solutions and mixed together at room temperature for 5-8 min before addition to the cells. Both green and red fluorescnces are measured versus time with a flow cytometer (FACS, Becton Dickinson). For each cell sample, the fluorescence of cells preloaded with the fluorescent Ca-probes is first recorded for 15 seconds in order to obtain the baseline values. The data acquisition is interrupted briefly to to stimulate the cells by addition of a small volume of stimulus (ten-fold concentrate of chemokine with or without antibody)and fluorecence measurements are resumed. The total fluorescence measurement lasts 51 seconds.

The $Ca^{2+}$ indicators used exhibit reciprocal shifts in fluorescence intensity on binding to calcium; fura red fluorescence decreases whereas fluo-3 fluorescence increases. For each experiment, red and green fluorescence are recorded and the ratios between green and red fluorescence calculated and plotted against time. A "base line ratio" and a "stimulation ratio" are respectively defined as the mean value of ratios obtained just before stimulation and the mean value of maximal ratios obtained after stimulation. The intensity of the response is quantified by a Stimulation Index (S.I.) given by "stimulation ratio" divided by "base line ratio". Stimulation Indices obtained in the presence of antibody are expressed as percentage of S.I. obtained in presence of solvent alone.

Inhibition (%) of an an antibody A1 dissolved in solvent S1 is given by the formula:

$$100-[S.I._{A1} \times 100)/S.I._{S1}]$$

The inhibition by ABN912 is compared to that of the precursor antibody, AAV293, and an unrelated commercially available anti-MCP-1 murine antibody (R&D Systems). Also since the immunogen used to raise AAV293 was unglycosylated recombinant human MCP-1 from E. coli, MCP-1 supernatant from TNF$\alpha$-stimulated HUVEC (Human Umbilical Vein Endothelial Cells) is also used to stimulate CHO#84 cells and the antagonising effect of ABN912 on $Ca^{2+}$ mobilisation measures as described above. The results obtained are given in the Table below

| Human MCP-1 | | Anti-human MCP-1 mAb | | |
|---|---|---|---|---|
| Source | Molarity (nM) | Human ABN912 (IC$_{50}$ in nM) | Human AAV293 (IC$_{50}$ in nM) | Murine R&D Systems (IC$_{50}$ in nM) |
| E. coli (unglycosylated) | 0.3<br>1<br>3 | $0.1 \pm 0.02$<br>$0.39 \pm 0.02$<br>$1.53 \pm 0.47$ | $0.20 \pm 0.00$<br>$0.66 \pm 0.16$<br>$2.12 \pm 0.06$ | $0.73 \pm 0.09$ |
| HUVEC (glycosylated) | 1 | $0.33 \pm 0.01$ | $0.73 \pm 0.04$ | $0.95 \pm 0.49$ |
| ABN912 specifically inhibits MCP-1 induced $Ca^{2+}$ mobilisation in CHO#84 cells, with similar potency for both E. coli derived and HUVEC derived MCP-1. | | | | |

**2.5 Inhibition of MCP-1 induced chemotaxis**

The ability of ABN912 to inhibit the chemotactic effect of MCP-1 on hPBMC (human Peripheral Blood Monocytic Cells) is measured in Boyden chamber based chemotaxis assays. HPBMC are prepared with Lymphoprep™ and 2 x

$10^6$ monocytes per ml are used as input. ABN912 or an unrelated antibody (negative control) is added at the indicated molar ratios in the bottom and top compartments of the chamber. Chemotaxis is induced with 5.7 nM recombinant human MCP-1 or 1 nM fMIFL (negative control) in the bottom chamber. Cells are permitted to migrate from the top chamber to the bottom chamber for a period of 90 minutes at room temperature. The number of cells which have migrated is determined by staining and counting. It is found that ABN912 dose dependently inhibits MCP-1 induced chemotaxis of hPBMCs. The effect of ABN912 on MCP-induced chemotaxis is specific; the unrelated antibody has no effect and ABN912 has no effect on fMIFL induced chemotaxis.

### 2.6 Inhibition of leukocyte emigration in rhesus monkeys

This mechanistic model was used to test whether MCP-1-induced emigration of leukocytes into the skin of rhesus monkeys can be inhibited by NVP-ABN912 when given as an i.v. bolus injection.

Male adult rhesus monkeys were injected with 30μg/kg IL-3 for 13 days (two times per day, i.v.) in order to boost leukocyte counts and prime the endothelial cells for optimal leukocyte recruitment. On day 13 the monkeys were anaesthetized and MCP-1 (10 μg) was injected in triplicates intradermally on the right side of the breast and abdomen. Four hours later, the monkeys were again anaesthetized and punch biopsies from the MCP-1 injection sites were taken. Then either NVP-ABN912 or an isotype matched humanized negative control antibody (CGP44290) directed against human carcinoembryonic antigen was given as i.v. bolus injection to reach a dose of 5mg/kg. Finally, MCP-1 (10μg) was injected again in triplicates intradermally on the left side of the breast and the abdomen. These injection sites were symmetrically positioned relative to the previous injection series. Four hours later, the monkeys were anaesthetized and punch biopsies from the MCP-1 injection sites were taken. All punch biopsies were then cut in half and one half frozen in liquid nitrogen for enzyme analyses. The second half was preserved in formalin for later histology. Enzyme assays for eosinophils and neutrophils were eosinophilperoxidase (EPO) activity and myeloperoxidase (MPO) activity, respectively. Controls with PBS injections and no injections to stimulate cell infiltration were run in parallel on each monkey. No significant antibody mediated effects were observed with either control.

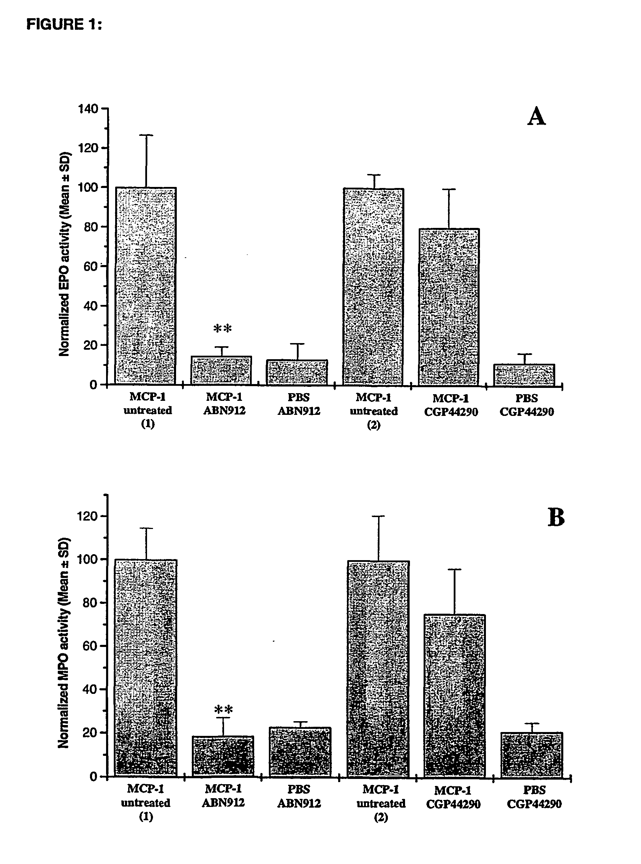

The enzymatic activities at MCP-1 injection sites before and after treatment with NVP-ABN912 and CGP44290 (IgG4, isotype matched negative control), respectively, are shown as mean $\pm$ SD. Multiple injection sites (3) were employed on every monkey for each condition. Combined data from two independently run experiments are shown in Figure 1. A-eosinophil emigration and Figure 1B neutrophil emigration.

Normalized EPO (A) or MPO (B) activities in punch biopsies before (MCP-1 untreated, 100%) and after treatment with antibodies are shown as mean $\pm$ SD.

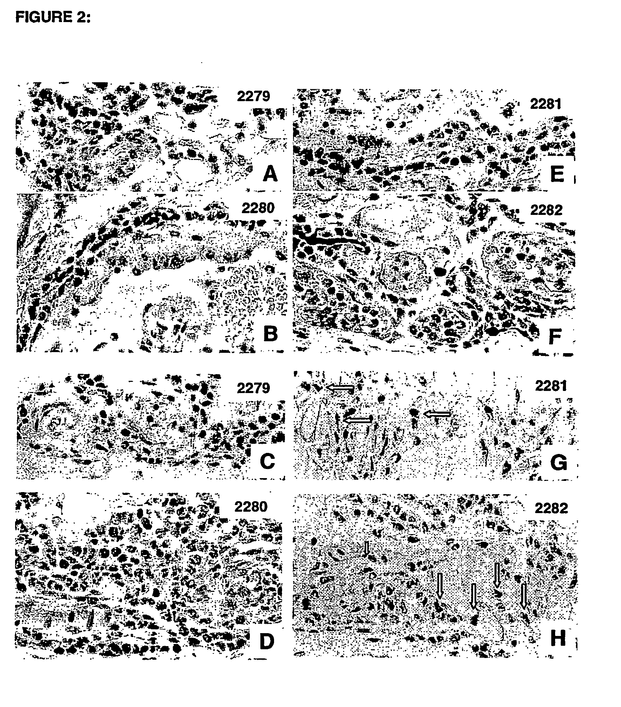

When leukocyte emigration was induced in rhesus monkey skin by 10 μg MCP-1, an inhibition of 85.5% of eosinophil and 81.4% of neutrophil emigration was observed in presence of NVP-ABN912 compared to the leukocyte emigration prior to antibody treatment. Only about 20% inhibition for both cell types was measured when CGP44290 was applied. Taking into account that a PBS injection alone resulted in a recruitment of 13.0% (eosinophils) and 22.7% (neutrophils) of the response seen with 10 μg MCP-1, administration of NVP-ABN912 reduced the amount of leukocyte emigration to the background level observed with the PBS stimulation. The differences observed between NVP-ABN912 and the control antibody are statistically highly significant (p<0.01) and considering the magnitude of the effect, it seems also of biological relevance. Representative histology of eosinophil emigration is shown in Figure 2.

Histology of punch biopsies from MCP-1-induced eosinophil emigration experiment is shown for four monkeys (2279, 2280, 2281 and 2282).
(A), (B), (E), (F) eosinophil staining before treatment with antibodies of monkeys 2279, 2280, 2281 and 2282, respectively.
(C), (D), (G), (H), eosinophil staining after treatment with antibodies of monkeys 2279, 2280, 2281 and 2282, respectively.
Monkeys 2279 and 2280 were treated with the isotype matched negative control antibody CGP44290 (5mg/kg) and monkeys 2281 and 2282 were treated with NVP-ABN912 (5mg/kg). Eosinophils are stained red (seen as dark spots in black and white) and arrows in panels (G) and (H) highlight the few eosinophils present in the two NVP-ABN912 treated monkeys.
No difference between before and after treatment was observed with CGP 44290 while an almost complete inhibition of eosinophil emigration was observed in monkeys treated with NVP-ABN912.

### 2.7 Inhibition of T cell infiltration in SCID-hu mice

This mechanistic model was used to test whether MCP-1-induced infiltration of Th cells into the skin of SCID-hu Skin mice can be inhibited by i.p. injection of NVP-ABN912.
SCID mice were transplanted with two small pieces of human adult skin (SCID-hu Skin). The quality of the grafts was monitored during the 5-6 weeks following transplantation and then, successfully transplanted mice (generally >85%) were selected for the *in vivo* migration experiments. For the chemokine-induced migration, PBMC were isolated by standard density gradient separation from buffy coats samples and adoptively transferred (i.p. 1x$10^8$ cells/mouse, 500

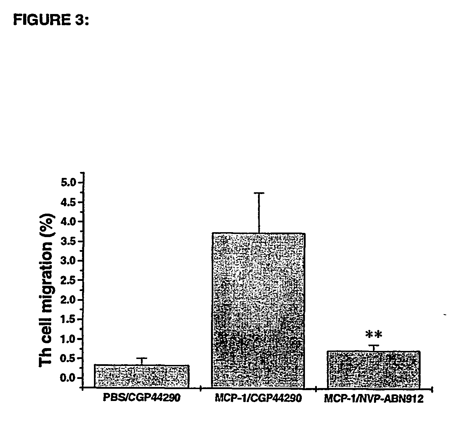

μl volume) into SCID-hu Skin mice which were previously transplanted (5-8 weeks) with two pieces of human skin (at the right and left sides of the upper back). Cell transfer was done at day 0. MCP-1 (R&D Systems, Minneapolis, MN) and PBS were administered intracutaneously into the human grafts on experimental days 1, 2, 4 and 6. Control mice (CGP44290 treated) received 500 ng MCP-1 in the right skin graft and an equal volume (30-μl) of PBS in the left graft. Mice treated with NVP-ABN912 were injected with 500 ng MCP-1 in both, right and left skin grafts. NVP-ABN912 and the isotype control CGP44290 were administered i.p. (100 μg/mouse, 4mg/kg, 500 μl volume) at day 0 (5h before cell transfer) and at experimental days 2 and 5. On day 8, all mice were sacrificed and the human skin grafts harvested for further analysis. Single cell suspensions of each human skin graft were prepared using a DAKO Medimachine® following the manufacturer's instructions. These cell suspensions were stained with human anti-CD3 and anti-CD4 mAb conjugated to FITC and PE and analyzed in a FACSCalibur Flow Cytometer (Becton Dickinson, San Jose, CA). The results are given in Figure 3. 0.73 $\pm$ 0.15% of infused human Th cells infiltrated the skin graft in the ABN912 treated group (5 mice, n = 10) compard to 3.73 $\pm$ 1.03% found in the corresponding CPG44290 treated group (3 mice, n = 3). In mice where the cell migration was induced by PBS, 0.34 $\pm$ 0.17% of the Th cells infiltrated the human skin graft (3 mice, n = 3). Statistical analysis was done with one-way analysis of variance followed by Dunnett's multiple comparisons test *post hoc.* The reduction of Th cell infiltration in ABN912 versus CGP44290 treated animals, respectively, was highly significant (p<0.01, **).

**2.8 Characterisation of the ABN912 binding epitope on human MCP-1 and mode of action**

The three-dimensional structure of the complex of MCP-1 with the Fab fragment of NVP-ABN912 was determined by X-ray crystallography.
The Fab fragment of NVP-ABN912 was produced by proteolytic cleavage from the whole antibody and purified by protein A chromatography followed by size-exclusion chromatography. The complex between the Fab and recombinant human MCP-1 was purified by protein G and size-exclusion chromatography, and concentrated by ultrafiltration to 26 mg/ml in 50 mM Tris-HCl pH 8.0, 0.1M NaCl. Crystals were grown at room temperature by the technique of vapor diffusion in hanging drops, in 20% (w/v) PEG 4,000, 10% (v/v) isopropanol, 0.1M Na Hepes pH 7.5. They were in space group $P2_12_12_1$ with unit cell dimensions a=63.90Å, b=86.08Å, c=321.64Å and three complexes per asymmetric unit. Prior to data collection, one crystal was soaked in 17% (w/v) PEG 4,000, 8.5% (v/v) isopropanol, 15% glycerol, 85mM Na Hepes pH 7.5 for about 3 minutes. The crystal was then mounted in a nylon CryoLoop and directly frozen in a nitrogen stream at 120K. The diffraction data were measured with a MAR 345 image plate system at the SNBL beamline of the European Synchrotron Radiation Facility ($\lambda$ =0.90 Å). In total 242,617 observations, corresponding to 68,948 unique reflections (Rsym=0.050), were collected between 20.0 and 2.33Å resolution (89.3% completeness). The structure was determined by molecular replacement, using the X-ray structures of the Fab fragment of the 3D6 monoclonal antibody (RCSB entry 1DFB; He et al., 1992) and of human MCP-1 (RCSB entry IDOL; Lubkowski et al., 1997) as starting models. The structure was refined by torsion angle dynamics and energy minimization using the program CNX, to a final R-factor of 0.224 (Rfree=0.261) for all reflections between 20 and 2.33Å. The final model includes L1 to L214 and H1 to H222 of ABN912 and residues 10 to 71 of human MCP-1. It has good geometry, with an rms deviation of 0.006Å on bond lengths and 1.36° on bond angles.
The NVP-ABN912 Fab/human MCP-1 complex shows a large binding interface (1,590Å$^2$ of combined buried surface) involving many hydrophobic and polar interactions as well as several key electrostatic interactions. The major contacts to the antigen are mediated by the long H-CDR3 loop of NVP-ABN912 which folds over the center of the antigen-combined site and becomes largely buried in the complex. The binding epitope on human MCP-1 comprises the amino acid residues Asn 14, Thr 16, Asn 17, Arg 18, Lys 19, Ile 20, Ser 21, Gln 23, Arg 24, Lys 49, Glu 50, Ile 51 and Cys 52. Arg 18, Arg 24 and Lys 49 are involved in electrostatic interactions with the antibody residues Glu L55 , Asp H99 and Glu H101, and in H-bonded interactions with Tyr L94, Trp H33, Asn H50, Gln H53, Asp H99 and Tyr H108. In addition, the guanidinium moiety of Arg 18 and Arg 24 of MCP-1 are π -stacked against the aromatic rings of Trp H33 and Tyr H32, respectively. Additional interactions between NVP-ABN912 and Tyr 13, Gln 17, Ser 21, Lys 19, Arg 24, and Glu 50 of the antigen are mediated by eight water molecules buried in the protein interface. Since Tyr 13 and Arg 24 of MCP-1 have been implicated in the binding to the CCR2B chemokine receptor (Hemmerich et al., 1999, Biochemistry, 38; 13013-13025, it appears that NVP-ABN912 directly competes with the receptor for antagonist binding.

**2.9 Mapping of the ABN912 epitope on MCP-1 by site-directed mutagenesis**

In order to determine functionally important residues on MCP-1 for recognition by NVP-ABN912, an alanine-scanning mutagenesis study was performed (Cunningham, B.C. and Wells, J.A. (1989) Science 244, 1081-1085). First, the three-dimensional structure of MCP-1 (Handel, T.M. and Domaille, P.J. (1996) Biochemisty 35, 6569-6584; Lubkowski, J., Bujacz, G., Boqúe, L., Domaille, P.J., Handel, T.M. and Wlodawer, A. (1997) Nature Struct. Biol. 4, 64-69) was visually inspected to identify surface exposed residues using the software WebLab Viewer. A total of 39 residue that could potentially interact with ABN912 were individually mutated to alanine or lysine (D3K, A48K) with the QuikChange Site-Directed Mutagenesis kit (Papworth, C., Bauer, J.C., Braman, J. and Wright, D.A. (1996) Strategies 9 (3), 3-4). The resulting mutant genes were expressed in HEK.EBNA cells by first transfecting the cells with two micrograms of expression plasmids carrying the MCP-1 mutant sequences with the Geneporter transfection reagent (Gene Therapy Systems). After transfection, the cells were incubated for three days at 37°C and the supernatants were then collected, centrifuged for 5' and subjected to purification by affinity chromatography. Mutant MCP-1 concentrations were determined using the Protein Assay (Biorad) with purified MCP-1 as standard. Typically 40μg of purified human MCP-1 or MCP-1 mutants were obtained from 3ml of culture.

The affinity to NVP-ABN912 of MCP-1 and mutant MCP-1 was measured by surface plasmon resonance with the BIAcore instrument (BIAcore). First, the sensorchip surface of the instrument was activated and a 30 μg/ml anti-Fcγ solution was injected to covalently bind to the chip. The NVP-ABN912 antibody was accumulated on the anti-Fcγ modified surface by injecting a 5μg/ml solution. Dilutions of MCP-1 or MCP-1 mutants were prepared to yield final concentrations of 0.75 to 4nM and injected to bind to the immobilized NVP-ABN912 on the sensorchip surface. Association and dissociation were both followed for 5 min during which the surface plasmon resonance signal was recorded. The titration series was then analyzed using the BIAevaluation 3.0 software (Software part of the instrument installation; Handbook Cat.No. BR-1002-29; edition 7-97). The results are given in Figure 3 which shows binding of MCP-1 and MCP-1 mutants to NVP-ABN912.

The main determinants of MCP-1 for NVP-ABN912 recognition, identified as described above, are the residues T16, R18, R24 and K49. Mutation of T16, R18 and R24 to alanine completely abolished binding to NVP-ABN912, while the mutation of K49 to alanine resulted in a 133-fold decrease of affinity.

**Claims**

1. A human monocyte chemo attractant protein (MCP-1) binding molecule comprising both heavy (V$_H$) and light chain (V$_L$) variable domains in which said MCP-1 binding molecule comprises at least one antigen binding site comprising:

   a) an immunoglobulin heavy chain variable domain (V$_H$) which comprises in sequence hypervariable regions cDR1, CDR2 and CDR3, said CDR1 having the amino acid sequence His-Tyr-Trp-Met-Ser, said CDR2 having amino acid sequence Asn-Ile-Glu-Gln-Asp-Gly-Ser-Glu-Lys-Tyr-Tyr-Val-Asp-Ser-Val-Lys-Gly, and said CDR3 having amino acid sequence Asp-Leu-Glu-Gly-Leu-His-Gly-Asp-Gly-Tyr-Phe-Asp-Leu, and
   b) an immunoglobulin light chain variable domain (V$_L$) which comprises in sequence hypervariable regions CDR1', CDR2' and CDR3', said CDR1' having the amino acid sequence Arg-Ala-Ser-Gln-Gly-Val-Ser-Ser-Ala-Leu-Ala, said CDR2' having the amino acid sequence Asp-Ala-Ser-Ser-Leu-Glu-Ser, and said CDR3' having the amino acid sequence Gln-Gln-Phe-Asn-Ser-Tyr-Pro;

2. An MCP-1 binding molecule according to claim 1, which is a human antibody.

3. An MCP-1 binding molecule which comprises at least one antigen binding site comprising a first domain having an amino acid sequence identical to that shown in Seq. Id. No. I starting with amino acid at position 1 and ending with amino acid at position 122 and a second domain having an amino acid sequence substantially identical to that shown in Seq. Id. No. 2, starting with amino acid at position I and ending with amino acid at position 109.

4. A first DNA construct encoding a heavy chain or fragment thereof which comprises

   a) a first part which encodes a variable domain comprising alternatively framework and hypervariable regions, said hypervariable regions being in sequence CDR1, CDR2 and CDR3 the amino acid sequences of which are shown in Seq. Id. No. I; this first part starting with a codon encoding the first amino acid of the variable domain and ending with a codon encoding the last amino acid of the variable domain, and
   b) a second part encoding a heavy chain constant part or fragment thereof which starts with a codon encoding the first amino acid of the constant part of the heavy chain and ends with a codon encoding the last amino acid of the constant part or fragment thereof, followed by a stop codon.

5. A second DNA construct encoding a light chain or fragment thereof which comprises

   a) a first part which encodes a variable domain comprising alternatively framework and hypervariable regions; said hypervariable regions being CDR1', CDR2' and CDR3', the amino acid sequences of which are shown in Seq. Id. No. 2; this first part starting with a codon encoding the first amino acid of the variable domain and ending with a codon encoding the last amino acid of the variable domain, and

b) a second part encoding a light chain constant part or fragment thereof which starts with a codon encoding the first amino acid of the constant part of the light chain and ends with a codon encoding the last amino acid of the constant part or fragment thereof followed by a stop codon.

6. An expression vector able to replicate in a prokaryotic or eukaryotic cell line which comprises at least one DNA construct according to claim 4 or claim 5.

7. A process for the production of an MCP-1 binding molecule with comprises (i) culturing an organism which is co-transformed with expression vectors according to claim 6 and (ii) recovering the MCP-1 binding molecule from the culture.

8. An antibody to MCP-1 of any one or the claims 1 to 3 which cross-reacts with eotaxin.

9. Use of an antibody to MCP-1 of any one of the claims 1 to 3 which cross-reacts with eotaxin which is capable of inhibiting the binding of MCP-1 and eotaxin to their receptors, for the preparation of a medicament for the treatment of an MCP-1 or eotaxin-mediated disease or disorder.

10. A pharmaceutical composition comprising of antibody to MCP-1 of any one of claims 1 to 3 which cross-reacts with eotaxin and which is capable of inhibiting the binding of MCP-1 and eotaxin to their receptors, in combination with a pharmaceutically acceptable excipient, diluent or carrier; and

11. An antibody to MCP-1 of any one of claims 1 to 3 which has a $K_D$ for binding to MCP-1 of about 50pM or less.

**Patentansprüche**

1. Menschliches MCP-1 (monocyte chemo attractant protein) bindendes Molekül, umfassend variable Domänen sowohl der schweren ($V_H$) als auch leichten Kette ($V_L$), wobei das MCP-1 bindende Molekül wenigstens eine Antigenbindungsstelle umfaßt, die folgendes umfaßt:

a) eine variable Domäne der schweren Immunglobulinkette ($V_H$), die aufeinanderfolgend die hypervariablen Bereiche CDR1, CDR2 und CDR3 umfaßt, wobei CDR1 die Aminosäuresequenz His-Tyr-Trp-Met-Ser, CDR2 die Aminosäuresequenz Asn-Ile-Glu-Gln-Asp-Gly-Ser-Glu-Lys-Tyr-Tyr-Val-Asp-Ser-Val-Lys-Gly und CDR3 die Aminosäuresequenz Asp-Leu-Glu-Gly-Leu-His-Gly-Asp-Gly-Tyr-Phe-Asp-Leu aufweist, und
b) eine variable Domäne der leichten Immunglobulinkette ($V_L$), die aufeinanderfolgend die hypervariablen Bereiche CDR1', CDR2' und CDR3' umfaßt,

wobei CDR1' die Aminosäuresequenz Arg-Ala-Ser-Gln-Gly-Val-Ser-Ser-Ala-Leu-Ala, CDR2' die Aminosäuresequenz Asp-Ala-Ser-Ser-Leu-Glu-Ser und CDR3' die Aminosäuresequenz Gln-Gln-Phe-Asn-Ser-Tyr-Pro aufweist.

2. MCP-1 bindendes Molekül gemäß Anspruch 1, bei dem es sich um einen menschlichen Antikörper handelt.

3. MCP-1 bindendes Molekül, das wenigstens eine Antigenbindungsstelle umfaßt, die eine erste Domäne mit einer Aminosäuresequenz, die mit der in Seq. Id. No. 1 dargestellten Sequenz identisch ist, beginnend mit der Aminosäure in Position 1 und endend mit der Aminosäure in Position 122, und eine zweite Domäne mit einer Aminosäuresequenz, die weitgehend zu der in Seq. Id. No. 2 dargestellten Sequenz identisch ist, beginnend mit der Aminosäure in Position 1 und endend mit der Aminosäure in Position 109, umfaßt.

4. Erstes DNA-Konstrukt, codierend für eine schwere Kette oder ein Fragment davon, das folgendes umfaßt:

a) einen ersten Teil, der für eine variable Domäne codiert, die alternativ Gerüst- und hypervariable Bereiche umfaßt, wobei es sich bei den hypervariablen Bereichen aufeinanderfolgend um CDR1, CDR2 und CDR3 handelt, deren Aminosäuresequenzen in Seq. Id. No. 1 dargestellt sind, wobei dieser erste Teil mit einem für die erste Aminosäure der variablen Domäne codierenden Codon beginnt und mit einem für die letzte Aminosäure der variablen Domäne codierenden Codon endet, und
b) einen zweiten Teil, der für einen konstanten Teil der schweren Kette oder ein Fragment davon codiert, der mit einem für die erste Aminosäure des konstanten Teils der schweren Kette codierenden Codon beginnt und mit einem für die letzte Aminosäure des konstanten Teils oder Fragments davon codierenden Codon endet, gefolgt von einem Stopcodon.

5. Zweites DNA-Konstrukt, codierend für eine leichte Kette oder ein Fragment davon, das folgendes umfaßt:

a) einen ersten Teil, der für eine variable Domäne codiert, die alternativ Gerüst- und hypervariable Bereiche umfaßt, wobei es sich bei den hypervariablen Bereichen um CDR1', CDR2' und CDR3' handelt, deren Aminosäuresequenzen in Seq. Id. No. 2 dargestellt sind, wobei dieser erste Teil mit einem für die erste Aminosäure der variablen Domäne codierenden Codon beginnt und mit einem für die letzte Aminosäure der variablen Domäne codierenden Codon endet, und
b) einen zweiten Teil, der für einen konstanten Teil der leichten Kette oder ein Fragment davon codiert, der mit einem für die erste Aminosäure des konstanten Teils der leichten Kette codierenden Codon beginnt und mit einem für die letzte Aminosäure des konstanten Teils oder Fragments davon codierenden Codon endet, gefolgt von einem Stopcodon.

6. Expressionsvektor, der zur Replikation in einer prokaryontischen oder eukaryontischen Zellinie fähig ist und der wenigstens ein DNA-Konstrukt gemäß Anspruch 4 oder Anspruch 5 umfaßt.

7. Verfahren zur Herstellung eines MCP-1 bindenden Moleküls, bei dem man (i) einen Organismus, der mit Expressionsvektoren gemäß Anspruch 6 cotransformiert ist, kultiviert und (ii) das MCP-1 bindende Molekül aus der Kultur gewinnt.

8. Antikörper gegen MCP-1 nach einem der Ansprüche 1 bis 3, der mit Eotaxin kreuzreagiert.

9. Verwendung eines Antikörpers gegen MCP-1 nach einem der Ansprüche 1 bis 3, der mit Eotaxin kreuzregiert und in der Lage ist, die Bindung von MCP-1 und Eotaxin an deren Rezeptoren zu hemmen, zur Herstellung eines Arzneimittels für die Behandlung einer durch MCP-1 oder Eotaxin vermittelten Krankheit oder Störung.

10. Pharmazeutische Zusammensetzung, umfassend den Antikörper gegen MCP-1 nach einem der Ansprüche 1 bis 3, der mit Eotaxin kreuzreagiert und in der Lage ist, die Bindung von MCP-1 und Eotaxin an deren Rezeptoren zu hemmen, in Kombination mit einem pharmazeutisch unbedenklichen Hilfsstoff, Verdünnungsmittel oder Trägerstoff; und

11. Antikörper gegen MCP-1 nach einem der Ansprüche 1 bis 3, der einen $K_D$-Wert für die Bindung an MCP-1 von etwa 50 pM oder weniger aufweist.

## Revendications

1. Molécule de liaison d'une protéine chimioattractive monocytaire humaine (MCP-1) comprenant à la fois les domaines variables de chaîne lourde ($V_H$) et de chaîne légère ($V_L$), dans laquelle ladite molécule de liaison de la MCP-1 comprend au moins un site de liaison à l'antigène comprenant :

a) un domaine variable de chaîne lourde d'immunoglobuline ($V_H$) qui comprend dans l'ordre les régions hypervariables CDR1, CDR2 et CDR3, ladite CDR1 présentant la séquence d'acides aminés His-Tyr-Trp-Met-Ser, ladite CDR2 présentant la séquence d'acides aminés Asn-Ile-Glu-Gln-Asp-Gly-Ser-Glu-Lys-Tyr-Tyr-Val-Asp-Ser-Val-Lys-Gly, et ladite CDR3 présentant la séquence d'acides aminés Asp-Leu-Glu-Gly-Leu-His-Gly-Asp-Gly-Tyr-Phe-Asp-Leu, et
b) un domaine variable de chaîne légère d'immunoglobuline ($V_L$) qui comprend dans l'ordre les régions hypervariables CDR1', CDR2' et CDR3', ladite CDR1' présentant la séquence d'acides aminés Arg-Ala-Ser-Gln-Gly-Val-Ser-Ser-Ala-Leu-Ala, ladite CDR2' présentant la séquence d'acides aminés Asp-Ala-Ser-Ser-Leu-Glu-Ser, et ladite CDR3' présentant la séquence d'acides aminés Gln-Gln-Phe-Asn-Ser-Tyr-Pro.

2. Molécule de liaison de la MCP-1 selon la revendication 1, qui est un anticorps humain.

3. Molécule de liaison de la MCP-1 qui comprend au moins un site de liaison à l'antigène, comprenant un premier domaine présentant une séquence d'acides aminés identique à celle montrée dans la Seq. Id. No. 1 commençant par l'acide aminé en position 1 et se terminant par l'acide aminé en position 122 et un deuxième domaine présentant une séquence d'acides aminés substantiellement identique à celle montrée dans la Seq. Id. No. 2, commençant par l'acide aminé en position 1 et se terminant par l'acide aminé en position 109.

4. Première construction d'ADN codant pour une chaîne lourde ou un fragment de celle-ci qui comprend

a) une première partie qui code pour un domaine variable comprenant, en alternance, des régions de cadre et hypervariables, lesdites régions hypervariables étant, dans l'ordre, CDR1, CDR2 et CDR3 dont les séquences d'acides aminés sont représentées dans la Seq. Id. No. 1 ; cette première partie commençant par un codon codant pour le premier acide aminé du domaine variable et se terminant par un codon codant pour le dernier acide aminé du domaine variable, et
b) une deuxième partie codant pour une partie constante de chaîne lourde ou un fragment de celle-ci, qui démarre par un codon codant pour le premier acide aminé de la partie constante de la chaîne lourde et se terminant par un codon codant pour le dernier acide aminé de la partie constante ou d'un fragment de celle-ci, suivie d'un codon stop.

5. Deuxième construction d'ADN codant pour une chaîne légère ou un fragment de celle-ci qui comprend

a) une première partie qui code pour un domaine variable comprenant, en alternance, des régions de cadre et hypervariables, lesdites régions hypervariables étant CDR1', CDR2' et CDR'3 dont les séquences d'acides aminés sont représentées dans la Seq. Id. No. 2 ; cette première partie commençant par un codon codant pour le premier acide aminé du domaine variable et se terminant par un cordon codant pour le dernier acide aminé du domaine variable, et
b) une deuxième partie codant pour une partie constante de chaîne légère ou un fragment de celle-ci, qui démarre par un codon codant pour le premier acide aminé de la partie constante de la chaîne légère et se terminant par un codon codant pour le dernier acide aminé de la partie constante ou d'un fragment de celle-ci, suivie d'un codon stop.

6. Vecteur d'expression capable de se répliquer dans une lignée cellulaire procaryote ou eucaryote, qui comprend au moins une construction d'ADN selon la revendication 4 ou la revendication 5.

7. Procédé pour la production d'une molécule de liaison de la MCP-1 qui comprend (i) la culture d'un organisme qui est co-transformé avec des vecteurs d'expression selon la revendication 6 et (ii) la récupération de la molécule de liaison de la MCP-1 de la culture.

8. Anticorps contre MCP-1 selon l'une quelconque des revendications 1 à 3 qui réagit de manière croisée avec l'éotaxine.

9. Utilisation d'un anticorps contre la MCP-1 selon l'une quelconque des revendications 1 à 3 qui réagit de manière croisée avec l'éotaxine, qui est capable d'inhiber la liaison de la MCP-1 et de l'éotaxine à leurs récepteurs, pour la préparation d'un médicament destiné au traitement d'une maladie ou d'un trouble provoqué(e) par la MCP-1 ou l'éotaxine.

10. Composition pharmaceutique comprenant un anticorps contre la MCP-1 selon les revendications 1 à 3 qui réagit de manière croisée avec l'éotaxine et qui est capable d'inhiber la liaison de la MCP-1 et de l'éotaxine à leurs récepteurs, en combinaison avec un excipient, un diluant ou un support pharmaceutiquement acceptable.

11. Anticorps contre la MCP-1 selon l'une quelconque des revendications 1 à 3 qui présente un $K_D$ pour la liaison à la MCP-1 d'environ 50 pM ou moins.

FIGURE 1:

**FIGURE 2:**

FIGURE 3:

**FIGURE 4:**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 05276986 B
- JP 09067399 B
- JP 11060502 B
- EP 0546073 B1
- US P5545806 A
- US P5569825 A
- US P5625126 A
- US P5633425 A
- US P5661016 A
- US P5770429 A
- EP 0438474 B1
- EP 0463151 B1
- WO 9007861 A
- WO 881649 A
- EP 0256055 B
- EP 0323997 B
- EP 0338841 B

### Non-patent literature cited in the description

- **KABAT E.A. et al.** Sequences of Proteins of Immunological Interest. US Department of Health and Human Services, Public Health Service, National Institute of Health
- **KARLSSON et al.** *J. Immunol. Meth.,* 1991, vol. 145, 229-240
- **FISHWILD et al.** *Nat Biotechnol.,* 1996, vol. 14, 845-851
- **HEINRICH et al.** *J. Immunol.,* 1989, vol. 143, 3589-97
- **HEMMERICH et al.** *Biochemistry,* 1999, vol. 38, 13013-13025
- **CUNNINGHAM, B.C. ; WELLS, J.A.** *Science,* 1989, vol. 244, 1081-1085
- **HANDEL, T.M. ; DOMAILLE, P.J.** *Biochemisty,* 1996, vol. 35, 6569-6584
- **LUBKOWSKI, J. ; BUJACZ, G. ; BOQÚE, L. ; DOMAILLE, P.J. ; HANDEL, T.M. ; WLODAWER, A.** *Nature Struct. Biol.,* 1997, vol. 4, 64-69
- **PAPWORTH, C. ; BAUER, J.C. ; BRAMAN, J. ; WRIGHT, D.A.** *Strategies,* 1996, vol. 9 (3), 3-4